# EUROPÄISCHE PATENTANMELDUNG

(11) **EP 2 085 081 A1**
(43) Veröffentlichungstag der Anmeldung: **05.08.2009**
(21) Anmeldenummer: 08002013.4
(22) Anmeldetag: 04.02.2008
(51) Int. Cl.: A61K 31/137, A61P 25/02

(54) **3-(2-Dimethylaminomethyl-cyclohexyl)-phenol gegen polyneuropathischen Schmerz**

(71) Anmelder: Grünenthal GmbH, 52078 Aachen (DE)
(72) Erfinder: Christoph, Thomas Dr., 52080 Aachen (DE)
(74) Vertreter: Bülle, Jan

(57) **Zusammenfassung**

Die Erfindung betrifft die Verwendung des Wirkstoffs 3-(2-Dimethylaminomethyl-cyclohexyl)-phenol oder eines seiner pharmazeutisch verträglichen Salze zur Herstellung einer pharmazeutischen Zusammensetzung zur Behandlung von polyneuropathischem Schmerz, insbesondere bei diabetischer Polyneuropathie.

## Beschreibung

Die Erfindung betrifft die Behandlung von polyneuropathischem Schmerz, insbesondere bei diabetischer Polyneuropathie oder chemisch induzierter Polyneuropathie, durch Verabreichung von 3-(2-Dimethylaminomethyl-cyclohexyl)-phenol, vorzugsweise von (1 R,2R)-3-(2-Dimethylaminomethyl-cyclohexyl)-phenol, oder einem seiner pharmazeutisch verträglichen Salze.

Unter Neuropathie versteht man eine Erkrankung des Nervensystems. Bei der Mononeuropathie sind einzelne Nerven betroffen. Typische Mononeuropathien sind periphere Fazialisparese, Karpaltunnelsyndrom, Ulnarisläsion, Radialisparese und Peronäusparese. Bei der Polyneuropathie sind hingegen viele Nerven gleichzeitig betroffen. Etwa 3% aller Menschen über 60 Jahren leiden unter einer Polyneuropathie.

In Abhängigkeit von der jeweiligen Ursache können bei der Polyneuropathie motorische, sensible oder auch vegetative Nerven betroffen sein.

Es gibt zwei Hauptformen: periphere Polyneuropathie und autonome Polyneuropathie.

Die periphere Polyneuropathie betrifft das durch den Willen beeinflussbare Nervensystem, beispielsweise die Nerven, die für den Tastsinn (sensibel) oder die Muskelbewegung (motorisch) verantwortlich sind. Insgesamt sind Beeinträchtigungen der sensiblen Nervenfasern, die Informationen von der Peripherie zu Rückenmark und Gehirn leiten, früher und ausgeprägter zu beobachten. Die motorischen Nerven, die für die Bewegung der Muskeln verantwortlich sind, können aber auch betroffen sein. Muskelkrämpfe sind häufig, massive Lähmungen eher selten.

Die autonome Polyneuropathie zieht das unwillkürliche, nicht dem eigenen Willen gehorchende Nervensystem (Sympathikus und Parasympathikus) in Mitleidenschaft. Dieses vegetative Nervensystem reguliert beispielsweise das wechselnde Tempo des Herzschlags oder die Magen-Darmbewegungen bei der Verdauung. Bis zu 50 Prozent der Diabetiker leiden nach 20-jähriger Krankheit an einer autonomen Polyneuropathie. Die Beschwerden sind davon abhängig, welche Organe des Körpers betroffen sind:

Die Erkrankung kann sowohl die Isolierung des Nerven (Myelin) als auch den Zellfortsatz (Axon) betreffen. Es können symmetrische und asymmetrische Formen auftreten. Die Symptome sind je nach betroffenem Nervenfasertyp und Körperregion vielfältig. Da die Zellkörper (Somata) der sensiblen Nervenzellen in den Ganglien nahe am Rückenmark liegen und die Nervenfortsätze von dort aus versorgt werden, nehmen die längsten Nervenfasern, welche bis in die Zehen vom Organismus versorgt werden müssen, am ehesten Schaden. Häufig beginnt die Erkrankung mit unangenehmen Missempfindungen in den Zehen. Schreitet die Erkrankung fort, so wird die Verteilung der Missempfindungen von den Patienten häufig als "handschuh-" bzw. "sockenförmig" beschrieben. Die betroffenen Körperbereiche können kribbeln und unangenehm störend taub sein, schwerere Formen äußern sich in polyneuropathischem Schmerz, welcher häufig als brennender Schmerz empfunden wird. Es können ferner Fehlempfindungen, wie z.B. Kälte-, Hitze- oder Schwellungsgefühl auftreten.

Die Zeichen und Symptome der Polyneuropathie können in drei Gruppen unterteilt werden: (i) Small-Fiber sensorisch (z.B. brennende Schmerzen, cutane Hyperästhesie, Praästhesien, Lanzierende Schmerzen, Verlust der Schmerz- und Temperaturwahrnehmung, Verlust des viszeralen Schmerzempfindens, Fußulzeration), (ii) Large-Fiber sensorisch (z.B. Verlust der Vibrationswahrnehmung, Ataxie durch Verlust der Propiozeption, Verlust der Reflexe, verlangsamte Nervenleitgeschwindigkeit) und (iii) autonomisch (z.B. Herzrythmusstörungen, Ruhetachykardie, Verlust der adäquaten Herzfrequenzanpassung, stumme Herzinfarkte, Herzinsuffizienz, orthostatische Hypotension, gustatorisches Schwitzen, Hyperthermie, Gastroparese, neuropathischer Durchfall, Obstipation, Blasendysfunktion, erektile Dysfunktion, retrograde Ejakulation).

Je nach betroffenem Nervenfasertyp kann man folgende Modalitäten unterscheiden:
- Fasertyp A-α (I) (13-20 µm, myelinisiert): Propriozeption der Gliedmaßen;
- Fasertyp A-β (II) (6-12 µm, myelinisiert): Propriozeption der Gliedmaßen, Vibration, Druck;
- Fasertyp A-δ (III) (1-5 µm, myelinisiert): mechanischer, scharfer, stechender Schmerz, intensiver Druck auf die Haut (über mechanische Nociceptoren) und extreme Temperatur (über thermische Nociceptoren);
- Fasertyp C (IV) (0,2-1,5 µm, unmyelinisiert): brennender, andauernder, diffuser Schmerz, temperaturausgelöster Schmerz, mechanischer brennender Schmerz über polymodale Nociceptoren bei hoher Intensität der thermalen bzw. chemischen Stimuli.

Eine zufrieden stellende Bekämpfung der Schmerzen bei Polyneuropathie durch Verabreichung herkömmlicher Analgetika nur eingeschränkt möglich (H. Chen et al., Mayo Clin Proc. 2004, 79, 1533-45; M. Namaka et al., Clin Ther. 2004, 26, 951-79). Plazebokontrollierte Studien zur medikamentösen Behandlung der Schmerzen bei Polyneuropathie haben folgende NNT-Werte ergeben (*Numbers Needed to Treat* ≡ Anzahl der Patienten, die mit dem jeweiligen Medikament behandelt werden müssen, damit ein Patient eine mehr als 50%ige Schmerzlinderung erfährt):

| | | |
|---|---|---|
| Diabetische Polyneuropathie | Wirkstoff | NNT |
| | Imipramin | 1,4 |
| | andere tricyclische Antidepressiva | 2,4 |
| | Oextromethorphan | 1,9 |
| | Carbamazepin | 3,3 |
| | L-Dopa | 3,4 |
| | Tramadol | 3,4 |
| | Gabapentin | 3,7 |
| | Capsaicin | 5,9 |
| | selektive Serotonin Wiederaufnahme Inhibitoren | 6,7 |
| | Mexiletin | 10,0 |
| Postherpetische Neuralgie | tricyclische Antidepressiva | 2,3 |
| | Oxycodon | 2,5 |
| | Gabapentin | 3,2 |
| | Capsaicin | 5,3 |

(vgl. S.H. Sindrup et al., Pain, 1999, 83, 389-400).

Die Wirksamkeit eines Arzneistoffs bei der Behandlung von Schmerz kann sich grundsätzlich in verschiedener Hinsicht äußern. Neben der Linderung der Schmerzen auf einer Schmerzskala kann mitunter z.B. auch eine Anhebung der durchschnittlichen Schmerzschwelle (*average pain threshold*) und/oder eine Verringerung der Anzahl der schmerzsensitiven Druckpunkte (*tender points*) beobachtet werden, was besonders vorteilhaft ist.

Der Erfindung liegt die Aufgabe zugrunde, Arzneimittel bereitzustellen, welche Vorteile gegenüber herkömmlichen Arzneimitteln haben. Die Arzneimittel sollten eine Behandlung von polyneuropathischem Schmerz ermöglichen und dabei eine möglichst große Schmerzlinderung bei möglichst geringen Nebenwirkungen bewirken.

Diese Aufgabe wird durch den Gegenstand der Patentansprüche gelöst.

Es wurde überraschend gefunden, dass der Wirkstoff 3-(2-Dimethylaminomethyl-cyclohexyl)-phenol, insbesondere sein (1 R,2R)-Stereoisomer, bzw. deren pharmazeutisch verträgliche Salze eine hervorragende Wirksamkeit bei der Linderung von polyneuropathischem Schmerz zeigen. So haben experimentelle Untersuchungen ergeben, dass die Wirksamkeit von (1 R,2R)-3-(2-Dimethylaminomethyl-cyclohexyl)-phenol bei polyneuropathischen Schmerz im Tiermodell etwa viermal größer ist als bei mononeuropathischem Schmerz. Da der Wirkstoff nur geringe Nebenwirkungen zeigt, werden dadurch Therapiemöglichkeiten eröffnet, welche erhebliche Vorteile gegenüber herkömmlichen Behandlungsmethoden haben.

(1 R,2R)-3-(2-Dimethylaminomethyl-cyclohexyl)-phenol ist auch unter dem INN "Faxeladol" bekannt und hat folgende Struktur:

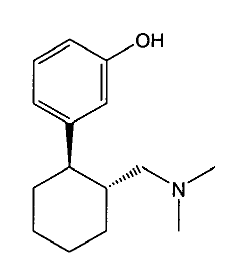

(1 R,2R)-3-(2-Dimethylaminomethyl-cyclohexyl)-phenol, einige seiner pharmazeutisch verträglichen Salze und die Verwendung zur Behandlung von bestimmten Schmerzarten sind aus dem Stand der Technik bekannt. In diesem Zusammenhang kann beispielsweise verwiesen werden auf DE-A 195 25 137, US 5,733,936, WO 2004/009067, WO 2004/ 047823 und DE 10 2005 034 974.

Die Erfindung betrifft die Verwendung des Wirkstoffs 3-(2-Dimethylaminomethyl-cyclohexyl)-phenol oder eines seiner pharmazeutisch verträglichen Salze zur Herstellung einer pharmazeutischen Zusammensetzung zur Behandlung von polyneuropathischem Schmerz.

Ein weiterer Aspekt der Erfindung betrifft 3-(2-Dimethylaminomethyl-cyclohexyl)-phenol oder eines seiner pharmazeutisch verträglichen Salze zur Behandlung von polyneuropathischem Schmerz.

Vorzugsweise liegt der Wirkstoff in Form des (1R,2R)-Stereoisomers, d.h. als (1R,2R)-3-(2-Dimethylaminomethyl-cyclohexyl)-phenol (Faxeladol) vor. Grundsätzlich sind aber auch die übrigen Stereoisomere, d.h. (1R,2S), (1S,2R) und (1S,2S) geeignet.

Der Wirkstoff kann als freie Base oder als pharmazeutisch verträgliches Salz vorliegen. Bevorzugte pharmazeutisch verträgliche Salze sind Salze anorganischer Säuren, z.B. das Hydrochlorid, Hydrobromid, Sulfat, Hydrogensulfat, Dihydrogenphosphat, Hydrogenphsophat und Phosphat; und Salze organischer Säuren, z.B. das Methansulfonat, Hexan-1-sulfonat, Formiat, Acetat, Oxalat, Succinat, Malat, Tartrat, Mandelat, Fumarat, Maleat, Lactat, Citrat, Glutamat, Saccharinat, Sebacinat, Monomethylsebacinat, 5-Oxo-Prolinat, Nicotinat, Benzoat, Aminobenzoat, Methylbenzoat, Trimethylbenzoat, α-Liponat, N-Acetylglycinat, N-Acetylalaninat, N-Acetylcysteinat, N-Acetylisoleucinat, N-Acetylleucinat, N-Acetylmethioninat, N-Acetylphenylalaninat, N-Acetylprolin, N-Acetylserin, N-Acetylthreonin, N-Acetyltyrosin, N-Acetylvalin, Acetylsalicylat, Acorbat, Hippurat und Asparaginat. Besonders bevorzugt liegt der Wirkstoff als Maleat vor.

Vorzugsweise ist der Wirkstoff in einer pharmazeutischen Zusammensetzung formuliert. Die erfindungsgemäße pharmazeutische Zusammensetzung kann z.B. fest, pastös oder flüssig sein. Vorzugsweise enthält sie pharmazeutisch verträgliche Hilfsstoffe, beispielsweise Füllmittel, Bindemittel, Lösungsmittel, Gleitmittel und/oder Sprengmittel. Die Wahl der jeweiligen Hilfsstoffe hängt davon ab, für welche Art der Verabreichung die pharmazeutische Zusammensetzung vorgesehen ist. Geeignete Hilfsstoffe sind dem Fachmann bekannt. In diesem Zusammenhang kann beispielsweise verwiesen werden auf H.P. Fiedler, Lexikon der Hilfsstoffe für Pharmazie, Kosmetik und angrenzende Gebiete, Editio Cantor Aulendorf.

Vorzugsweise liegt der Wirkstoffgehalt der pharmazeutischen Zusammensetzung im Bereich von 0,001 bis 99,999 Gew.-%, bevorzugter 0,1 bis 95 Gew.-%, noch bevorzugter 1,0 bis 80 Gew.-%, am bevorzugtesten 2,5 bis 65 Gew.-% und insbesondere 5,0 bis 50 Gew.-%, bezogen auf das Gesamtgewicht der pharmazeutischen Zusammensetzung.

Die erfindungsgemäße pharmazeutische Zusammensetzung kann neben 3-(2-Dimethylaminomethyl-cyclohexyl)-phenol ggf. weitere Wirkstoffe enthalten, welche beispielsweise ausgewählt sein können aus der Gruppe bestehend aus selektiven Serotonin- und Norepinephrin-Wiederaufnahme-Inhibitoren, α₂δ-Liganden, tricyclischen Antidepressiva, Opioiden und anderen Analgetika. Vorzugsweise ist jedoch nicht gleichzeitig ein COX II-Inhibitor enthalten, vorzugsweise überhaupt kein weiterer Wirkstoff.

Ein weiterer Aspekt der Erfindung betrifft eine wie vorstehend beschriebene pharmazeutische Zusammensetzung zur Behandlung von polyneuropathischem Schmerz.

In einer bevorzugten Ausführungsform liegt die pharmazeutische Zusammensetzung als Darreichungsform vor. Die erfindungsgemäße Darreichungsform kann z.B. fest, pastös oder flüssig sein. Vorzugsweise ist die erfindungsgemäße Darreichungsform zur systemischen, parenteralen, topischen oder lokalen Verabreichung konfektioniert. Vorzugsweise ist die erfindungsgemäße Darreichungsform zur oralen oder buccalen Verabreichung konfektioniert. Es sind jedoch auch andere Verabreichungsformen möglich, beispielsweise zur buccalen, sublingualen, transmucosalen, rektalen, intralumbalen, intraperitonealen, transdermalen, intravenösen, intramuskulären, intraglutealen, intrakutanen und subkutanen Applikation.

Je nach Konfektionierung enthält die Darreichungsform vorzugsweise geeignete Zusatz- und/oder Hilfsstoffe. Geeignete Zusatz- und/oder Hilfsstoffe im Sinne der Erfindung sind alle dem Fachmann aus dem Stand der Technik bekannten Stoffe zur Erreichung galenischer Formulierungen. Die Auswahl dieser Hilfsstoffe sowie die einzusetzenden Mengen hängen davon ab, wie die Darreichungsform appliziert werden soll, d.h. oral, intravenös, intraperitoneal, intradermal, intramuskulär, intranasal, buccal oder lokal.

Für die orale Applikation eignen sich Zubereitungen in Form von Tabletten, Kautabletten, Dragees, Kapseln, Granulaten, Tropfen, Säften oder Sirupen, für die parenterale, topische und inhalative Applikation Lösungen, Suspensionen, leicht rekonstituierbare Trockenzubereitungen sowie Sprays. Eine weitere Möglichkeit sind Suppositorien zur rektalen Anwendung. Die Anwendung in einem Depot in gelöster Form, einer Trägerfolie oder einem Pflaster, gegebenenfalls unter Zusatz von die Hautpenetration fördernden Mitteln, sind Beispiele für geeignete perkutane Applikationsformen.

Die Darreichungsform kann als einfache Tablette und als überzogene Tablette (z.B. als Filmtablette oder Dragee) vorliegen. Die Tabletten sind üblicherweise rund und bikonvex, oblong Formen sind jedoch ebenfalls möglich. Granulate, Sphäroide, Pellets oder Mikrokapseln, welche in Sachets oder Kapseln gefüllt oder zu zerfallenden Tabletten verpresst sind, sind ebenfalls möglich.

Beispiele für Hilfs- und Zusatzstoffe für die oralen Applikationsformen sind Sprengmittel, Gleitmittel, Binder, Füllmittel, Formtrennmittel, gegebenenfalls Lösungsmittel, Geschmacksstoffe, Zucker, insbesondere Trägermittel, Verdünnungsmittel, Farbstoffe, Antioxidantien etc.

Für Suppositorien können u.a. Wachse bzw. Fettsäureester und für parenterale Applikationsmittel Trägerstoffe, Konservierungsmittel, Suspensionshilfsmittel etc. verwendet werden.

Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnussöl, Erdnussöl, Sojabohnenöl, Lecithin, Natriumlactat, Polyoxyethylen- und -propylenfettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Herstellung dieser Arzneimittel und pharmazeutischen Zusammensetzungen erfolgt mit Hilfe von im Stand der Technik der pharmazeutischen Technologie wohlbekannten Mitteln, Vorrichtungen, Methoden und Verfahren, wie sie beispielsweise in *"*Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammensetzung zu bilden, die den Wirkstoff in homogener Verteilung enthält. Unter einer homogenen Verteilung wird hier verstanden, dass der Wirkstoff gleichmäßig über die gesamte Zusammensetzung verteilt ist, so dass diese ohne weiteres in gleich wirksame Einheitsdosis-Formen, wie Tabletten, Kapseln, Dragees unterteilt werden kann. Die feste Zusammensetzung wird anschließend in Einheitsdosis-Formen unterteilt. Die Tabletten oder Pillen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

In einer bevorzugten Ausführungsform ist die erfindungsgemäße Darreichungsform zur ein-, zwei- oder dreimal täglichen Verabreichung konfektioniert.

Eine verzögerte Freisetzung des Wirkstoffs kann beispielsweise durch Retardierung mit Hilfe einer Matrix, eines Überzugs oder osmotisch wirkender Freisetzungssysteme erreicht werden (vgl. z.B. WO 2005/009329).

Bevorzugt setzt die erfindungsgemäße Darreichungsform unter *in vitro* Bedingungen nach 1 h wenigstens 5 Gew.-%, bevorzugter wenigstens 10 Gew.%, noch bevorzugter wenigstens 15 Gew.%, am bevorzugtesten wenigstens 20 Gew.-% und insbesondere wenigstens 25 Gew.-% des ursprünglich in der Darreichungsform enthaltenen Wirkstoffs frei. Bevorzugt setzt die erfindungsgemäße Darreichungsform unter *in vitro* Bedingungen nach 1 h höchstens 95 Gew.-%, bevorzugter höchstens 90 Gew.-%, noch bevorzugter höchstens 85 Gew.-%, am bevorzugtesten höchstens 80 Gew.-% und insbesondere höchstens 75 Gew.-% des ursprünglich in der Darreichungsform enthaltenen Wirkstoffs frei. Geeignete Methoden zur Bestimmung der *in vitro* Freisetzungsrate sind dem Fachmann bekannt. Vorzugsweise erfolgt die Bestimmung unter sink Bedingungen bei 75 U/min in einem Puffer (gemäß Ph. Eur.) bei einem pH-Wert von 6,8 bei 37°C und unter UV-spektroskopischer Detektion mit Hilfe einer Blattrührapparatur oder der Drehkörbchenmethode.

Die erfindungsgemäße Darreichungsform enthält den Wirkstoff vorzugsweise in einer Dosierung im Bereich von 1,0 bis 1000 mg, bevorzugter 5,0 bis 900 mg, noch bevorzugter 10 bis 800 mg, am bevorzugtesten 15 bis 700 mg und insbesondere 20 bis 600 mg, jeweils bezogen auf die freie Base. Üblicherweise werden 0,1 bis 5000 mg/kg, insbesondere 1 bis 500 mg/kg, vorzugsweise 2 bis 250 mg/kg Körpergewicht verabreicht. Ebenso bevorzugt und üblich ist aber auch die Verabreichung von 0,01 bis 5 mg/kg, vorzugsweise 0,03 bis 2 mg/kg, insbesondere 0,05 bis 1 mg/kg.

In einer bevorzugten Ausführungsform
- ist die Darreichungsform zur oralen Verabreichung konfektioniert; und/oder
- ist die Darreichungsform eine feste und/oder verpresste und/oder filmbeschichtete Arzneiform; und/oder
- setzt die Darreichungsform den Wirkstoff aus einer Matrix verzögert frei; und/oder
- enthält die Darreichungsform den Wirkstoff in einer Menge von 0,001 bis 99,999 Gew.-%, bevorzugter 0,1 bis 99,9 Gew.-%, noch bevorzugter 1,0 bis 99,0 Gew.-%, noch bevorzugter 2,5 bis 80 Gew.-%, am bevorzugtesten 5,0 bis 50 Gew.-% und insbesondere 7,5 bis 40 Gew.-%, bezogen auf das Gesamtgewicht der Darreichungsform; und/oder
- enthält die Darreichungsform einen pharmazeutisch verträglichen Träger und/oder pharmazeutisch verträgliche Hilfsstoffe; und/oder
- weist die Darreichungsform eine Gesamtmasse im Bereich von 25 bis 2.000 mg, bevorzugter 50 bis 1.800 mg, noch bevorzugter 60 bis 1.600 mg, noch bevorzugter 70 bis 1.400 mg, am bevorzugtesten 80 bis 1.200 mg und insbesondere 100 bis 1.000 mg auf, und/oder
- ist die Darreichungsform ausgewählt aus der Gruppe bestehend aus Tabletten, Kapseln, Pellets und Granulaten.

Ein weiterer Aspekt der Erfindung betrifft eine wie vorstehend beschriebene Darreichungsform zur Behandlung von polyneuropathischem Schmerz.

Der Wirkstoff 3-(2-Dimethylaminomethyl-cyclohexyl)-phenol und seine pharmazeutisch verträglichen Salze eignet sich zur Behandlung von polyneuropathischem Schmerz. Bevorzugt ist der Schmerz peripherer polyneuropathischer Schmerz oder zentraler polyneuropathischer Schmerz. Bevorzugt ist die Polyneuropathie bzw. der polyneuropathische Schmerz akut (bis vier Wochen), subakut (vier bis acht Wochen) oder chronisch (mehr als acht Wochen). Bevorzugt ist bei der Polyneuropathie das motorische, sensible, autonome, sensomotorische oder zentrale Nervensystem betroffen. Vorzugsweise sind die Symptome symmetrisch oder asymmetrisch verteilt. Der Schmerz kann leicht, mäßig, mittelstark, stark oder sehr stark sein. Als Maß kann die neuropathische Schmerzskala (NPS) dienen (vgl. B.S. Galer et al., Neurology 1997, 48, 332-8).

Beispiele für Ursachen von peripherem polyneuropathischem Schmerz sind diabetische Polyneuropathie, postherpetische Neuralgie, Radioculopathie, posttraumatische Neuralgie, durch chemische Substanzen, z.B. durch Chemotherapie induzierte Polyneuropathie, Phantomschmerzen an den Gliedmaßen, komplexes regionales Syndrom, HIV-induzierte sensorische Polyneuropathie und alkoholische Polyneuropathie. Beispiele für Ursachen von zentralem polyneuropathischem Schmerz sind kompressive Myelopathie infolge von verengter Kanalstenose, posstraumatischer Spinalschmerz, Schlaganfallschmerz, postischemische Myelopathie, strahleninduzierte Myelopathie, durch Multiple Sklerose induzierte Myelopahthie und HIV-induzierte Myelopathie.

In einer bevorzugten Ausführungsform ist die den polyneuropathischen Schmerz verursachende Polyneuropathie assoziiert mit einer Erkrankung ausgewählt aus der Gruppe bestehend aus Diabetes, Diabetes mellitus, Vaskulitis, Urämie, Hypotyrodismus, Alkoholmissbrauch, postherpetischer Neuralgie, idiopathischer Neuropahthie, chronisch entzündlicher demyelinisierender Neuropathie, multifokaler motorischer Neuropathie, hereditärer Polyneuropathie, Guillain-Barrä-Syndrom, Intoxikation [z.B. durch Alkohol, Schwermetalle {insbesondere Pb, Hg, As}, Hydrocarbone, infolge einer Chemotherapie mit Cytostatika], Porphyrie, Infektionskrankheiten, Krebserkrankungen [z.B. Myelom, Amyloid, Leukämie, Lymphome], perniziöse Anämie, Vitamin-E-Mangel, Morbus Refsum, Bassen-Kornzweig-Syndrom, Morbus-Fabry, Vaskulitis und Amyloidose. Diabetische Polyneuropathie und postherpetische Neuralgie sind besonders bevorzugt. Handelt es sich um eine Infektionskrankheit, so ist diese vorzugsweise ausgewählt aus der Gruppe bestehend aus Mononukleose, Ehrlichiose, Typhus, Diphterie, Lepra, HIV, Lues und Borreliose.

Bevorzugt handelt es sich bei dem polyneuropathischen Schmerz um Schmerz, welcher als Ursache eine Polyneuropathie im Sinne der ICD-10 hat (Internationale statistische Klassifikation der Krankheiten und verwandter Gesundheitsprobleme, WHO Ausgabe, vorzugsweise Stand 2008). Bevorzugt ist die Polyneuropathie ausgewählt aus paraneoplastischer Polyneuropathie, hereditärer und idiopathischer Neuropathie [G60], Polyneuritis [G61], sonstigen Polyneuropathien [G62], Polyneuropathie bei anderenorts klassifizierten Krankheiten [G63], Neuralgie o.n.A. [M79.2-], Neuritis o.n.A. [M79.2-], Peripherer Neuritis während der Schwangerschaft [026.83] und Radikulitis o.n.A. [M54.1-].

Handelt es sich um eine hereditäre oder idiopathische Neuropathie [G60], so ist diese vorzugsweise ausgewählt aus der Gruppe bestehend aus hereditärer sensomotorischer Neuropathie [G60.0] (Charcot-Marie-Tooth-Hoffmann-Syndrom, Dejerine-Sottas-Krankheit, hereditäre sensomotorische Neuropathie, Typ I-IV, hypertrophische Neuropathie des Kleinkindalters, Peronäale Muskelatrophie (axonaler Typ) (hypertrophische Form), Roussy-Lévy-Syndrom); Refsum-Krankheit [G60.1]; Neuropathie in Verbindung mit hereditärer Ataxie [G60.2]; idiopathischer progressiver Neuropathie [G60.3]; sonstiger hereditärer und idiopathischer Neuropathie [G60.8] (Morvan-Krankheit, Nélaton-Syndrom, Sensible Neuropathie: dominant vererbt oder rezessiv vererbt); und hereditärer und idiopathischer Neuropathie, nicht näher bezeichnet [G60.9].

Handelt es sich um eine Polyneuritis [G61], so ist diese vorzugsweise ausgewählt aus der Gruppe bestehend aus Guillain-Barré-Syndrom (Polyradikuloneuropathie) [G61.0] (akute (post-) infektiöse Polyneuritis); Serumpolyneuropathie [G61.1], sonstiger Polyneuritiden [G61.8] und Polyneuritis, nicht näher bezeichnet [G91.9].

Handelt es sich um eine sonstige Polyneuropathie [G62], so ist diese vorzugsweise ausgewählt aus der Gruppe bestehend aus arzneimittelinduzierter Polyneuropathie [G62.0], Alkohol-Polyneuropathie [G62.1], Polyneuropathie durch sonstige toxische Agenzien [G62.2], sonstiger näher bezeichneter Polyneuropathie [G62.8] (strahleninduzierte Polyneuropathie, Critical-illness-Polyneuropathie [G62.80], sonstige näher bezeichnete Polyneuropathie [G62.88]) und Polyneuropathie, nicht näher bezeichnet [G62.9] (Neuropathie o.n.A.).

Handelt es sich um eine Polyneuropathie bei anderenorts klassifizierten Krankheiten [G63], so ist diese vorzugsweise ausgewählt aus der Gruppe bestehend aus Polyneuropathie bei anderenorts klassifizierten infektiösen und parasitären Krankheiten [G63.0] (Polyneuropathie bei: Diphtherie [A36.8†], infektiöser Mononukleose [B27̃.†], Lepra [A30̃.†], Lyme-Krankheit [A69.2†], Mumps [B26.8†], nach Zoster [B02.2†], Spätsyphilis [A52.1†], Spätsyphilis, konnatal [A50.4†], Tuberkulose [A17.8t]); Polyneuropathie bei Neubildungen [G63.1] [COÖ-D48†]; diabetischer Polyneuropathie [G63.2] [E10̃-E14†, vierte Stelle .4]; Polyneuropathie bei sonstigen endokrinen und Stoffwechselkrankheiten [G63.3] [E00̃-E07†, E15̃-E16†, E20-⁻E34†, E70̃-E89†]; Polyneuropathie bei alimentären Mangelzuständen [G63.4] [E40̃-E64†]; Polyneuropathie bei Systemkrankheiten des Bindegewebes [G63.5] [M30̃-M35†]; Polyneuropathie bei sonstigen Krankheiten des Muskel-Skelett-Systems [G63.6] [M00̃-M25†, M40̃-M96†]; und Polyneuropathie bei sonstigen anderenorts klassifizierten Krankheiten [G63.8] (urämische Neuropathie [N18.8†]).

In einer besonders bevorzugten Ausführungsform ist der polyneuropathische Schmerz mit diabetischer Polyneuropathie [G63.2] assoziiert. Mehr als die Hälfte aller Diabetiker entwickelt eine Nervenschädigung (Polyneuropathie), wenn sie länger als zehn Jahre an Diabetes erkrankt sind. Nimmt man alle Typ-1 und Typ-2 Diabetiker zusammen, leiden etwa 30 Prozent darunter.

In einer anderen, besonders bevorzugten Ausführungsform ist der polyneuropathische Schmerz durch toxische Agenzien induziert (chemisch induzierte Polyneuropathie), vorzugsweise durch Arzneimittel (z.B. Chemotherapeutika) [G62.0] oder Alkohol [G62.1]. Es sind verschiedene toxische Agenzien bekannt, welche eine Polyneuropathie induzieren können. Beispiele für Arzneimittel sind Cisplatin, Didanosin, Stavudin und Zalcitabin.

Die nachfolgenden Beispiele illustrieren die Erfindung, sind jedoch nicht einschränkend auszulegen.

### Beispiele

Männliche Sprague Dawley Ratten (140-180 g, Janvier, Frankreich) wurden bei Standardbedingungen (6.00-18.00 Uhr Licht, 18.00 Uhr-6.00 Uhr Dunkelheit; 20-24°C Raumtemperatur, 35-70% relative Luftfeuchte; Leitungswasser und Standardfutter (ad libitum) in Gruppen zu fünf Tieren in Macrolon Typ 4 Käfigen gehalten.

### Vergleichsbeispiel - mononeuropathischer Schmerz

Die Untersuchung erfolgte gemäß Bennett et al., Pain, 1988, 33, 87-107.

Unter Pentobarbitainarkose (Narcocen, 60 mg/kg i.p., Merial GmbH, Deutschland) wurden unilateral vier lose Ligaturen des rechten nervus ischiaticus gesetzt. Die Tiere entwickelten an der vom geschädigten Nerv innervierten Pfote eine Überempfindlichkeit, die nach einer Erholungsphase von einer Woche über etwa vier Wochen mit Hilfe einer 4°C kalten Metallplatte quantifiziert wurde (Kälte-Allodynie). Die Tiere wurden für einen Zeitraum von 2 min. auf dieser Platte beobachtet und die Anzahl der Wegziehreaktionen der geschädigten Pfote wurde gemessen. Bezogen auf den Vorwert vor Substanzapplikation wurde die Substanzwirkung über einen Zeitraum von einer Stunde an vier Zeitpunkten (15, 30, 45, 60 min nach Applikation) bestimmt. Die Hemmung der Kälte-Allodynie zu den einzelnen Messzeitpunkten wurde in Prozent Wirkung zum individuellen Vortest (%MPE) ausgedrückt, wobei der Vortest 0% MPE und eine vollständige Hemmung (0 Wegziehreaktionen pro 2 min) 100% MPE entsprach. Die Gruppengröße betrug n=10. Die Signifikanz einer Substanzwirkung wurde auf Basis der prozentualen Hemmwerte gegen die Vehikelgruppe mittels zweifaktorieller Varianzanalyse und posthoc Analyse nach Bonferroni, der ED₅₀-Wert über lineare Regressionsanalyse für die einzelnen Messpunkte oder die Fläche unter der Kurve (AUC) bestimmt.

(1 R,2R)- 3-(2-Dimethylaminomethyl-cyclohexyl)-phenol (10-31,6 mg/kg i.p.) führte zu einer dosisabhängigen Hemmung der Kälte-Allodynie. Die minimal wirksame Dosis, bei der eine statistisch signifikante Hemmung erfolgte, lag bei 21,5 mg/kg i.p. Der maximale Effekt betrug 80% MPE 15 min nach Gabe von 31,6 mg/kg, der ED₅₀-Wert (95% VB) betrug 17,6 (14,1-21,4) mg/kg 30 min nach Applikation.

Die gemessenen Ergebnisse sind in nachfolgender Tabelle zusammengefasst (Hemmung der Kälte-Allodynie in % MPE; * < 0.05 gegen Vehikel; n.s. nicht signifikant gegen Vehikel):

| Dosis | (%MPE) | 15 min | 30 min | 45 min | 60 min |
|---|---|---|---|---|---|
| 10 | MW | 22,16 | 20,93 | 8,60 | 9,23 |
| | SEM | 9,14 | 10,08 | 7,05 | 7,74 |
| | Signifikanz | n.s. | n.s. | n.s. | n.s. |
| 21,5 | MW | 56,62 | 61,06 | 34,96 | 16,33 |
| | SEM | 8,65 | 7,25 | 10,01 | 11,37 |
| | Signifikanz | * | * | n.s. | n.s. |
| 31,6 | MW | 80,03 | 79,15 | 61,68 | 21,18 |
| | SEM | 10,96 | 7,25 | 10,16 | 9,10 |
| | Signifikanz | * | * | * | n.s. |

### Beispiel - polvneuropathischer Schmerz

Die Untersuchung erfolgte gemäß Authier et al., Neuroreport, 1999, 10, 965-8.

Ratten wurden an fünf Tagen (Tag 4, 6, 8, 10, 12) mit Vehikel (0,9% NaCl) oder Vincristin (200 µg/kg i.v.) behandelt (1 ml/kg), was zu einer kumulativen Vincristin-Dosis von 1 mg/kg führte. Drei Tage nach der letzten Vincristin Behandlung hatten die Tiere eine Überempfindlichkeit gegen Kälte (Kälte-Allodynie) entwickelt, die über einen Zeitraum von drei Wochen anhielt. Zum Test wurden die Tiere unter einer Plastikhaube auf einen Gitterrost gesetzt und nach Habituation wurde die Kälte-Allodynie quantifiziert. Dazu wurde ein Tropfen Aceton (10 µl) mittels einer Spritze und einen dünnen Plastikschlauch vorsichtig auf eine Hinterpfote aufgebracht. Die Anzahl der induzierten Wegziehreaktionen (Schütteln, Stampfen oder Lecken) wurde über einen Zeitraum von 30 sec aufgenommen. Die kumulative Anzahl von 5 Stimulierungen (im Abstand von je 5 min) wurde vor und zu verschiedenen Zeiten nach der Substanz- oder Vehikelgabe bestimmt. Im Vergleich zum jeweiligen Vortest (0% MPE) und zur wöchentlichen Vehikelkontrollgruppe wurde der prozentuale Hemmwert (% MPE) bestimmt (100% MPE = 0 Wegziehreaktionen). Die Gruppengröße betrug üblicherweise n=10. Die Signifikanz einer Substanzwirkung wurde auf Basis der prozentualen Hemmwerte gegen die Vehikelgruppe mittels zweifaktorieller Varianzanalyse und posthoc Analyse nach Bonferroni, der ED₅₀-Wert über lineare Regressionsanalyse für die einzelnen Messpunkte bestimmt.

(1R,2R)-3-(2-Dimethylaminomethyl-cyclohexyl)-phenol (Fexeladol) (10-21,5 mg/kg i.p.) führte zu einer dosisabhängigen Hemmung der Kälte-Allodynie. Die minimal wirksame Dosis, bei der eine statistisch signifikante Hemmung erfolgte, lag bei 4,64 mg/kg i.p. Der maximale Effekt betrug 74% MPE 30 min nach Gabe von 21,5 mg/kg, der ED₅₀-Wert (95% VB) betrug 5,7 (3,3-11,1). Die gemessenen Ergebnisse sind in nachfolgender Tabelle zusammengefasst (Hemmung der Kälte-Allodynie in % MPE; * < 0.05 gegen Vehikel; n.s. nicht signifikant gegen Vehikel):

| Dosis | (%MPE) | 30 min | 180 min |
|---|---|---|---|
| 10 | MW | -28,9 | -22,2 |
| | SEM | 23,8 | 23,0 |
| | Signifikanz | n.s. | n.s. |
| 2,15 | MW | 48,4 | -37,7 |
| | SEM | 8,0 | 19,3 |
| | Signifikanz | n.s. | n.s. |
| 4,64 | MW | 67,9 | 22,6 |
| | SEM | 5,3 | 12,2 |
| | Signifikanz | * | n.s. |
| 10 | MW | 58,7 | 20,8 |
| | SEM | 17,7 | 13,8 |
| | Signifikanz | n.s. | n.s. |
| 21,5 | MW | 74,2 | 15,2 |
| | SEM | 12,3 | 10,0 |
| | Signifikanz | * | n.s. |

Die vorstehenden experimentellen Ergebnisse belegen, dass (1 R,2R)-3-(2-Dimethylaminomethyl-cyclohexyl)-phenol (Faxeladol) bei mono- und polyneuropathischem Schmerz eine dosisabhängige Hemmung der Kälte-Allodynie bewirkt. Ein signifikanter Unterschied ist im Hinblick auf die Wirkstärke in beiden Tiermodellen zu beobachten - während im polyneuropathischen Schmerzmodell bereits eine signifikante Hemmung bei 4,64 mg/kg i.p. beobachtet wird, tritt im mononeuropathischen Schmerzmodell eine signifikante Hemmung erst bei 21,5 mg/kg i.p. auf, d.h. bei einer mehr als viermal höheren Dosierung. Ähnlich verhält sich der ED₅₀-Wert, der zum gleichen Zeitpunkt nach Verabreichung (30 min) im polyneuropatischen Schmerzmodell 5,7 mg/kg i.p. und im mononeuropathischen Schmerzmodell 17,6 mg/kg i.p. beträgt, also im polyneuropathischen Schmerzmodell um etwa einen Faktor 3 wirksamer ist.

Diese Daten belegen, dass (1 R,2R)-3-(2-Dimethylaminomethyl-cyclohexyl)-phenol (Faxeladol) bzw. seine pharmazeutisch verträglichen Salze für die Behandlung von polyneuropathischem Schmerz in besonderer Weise geeignet ist.

## Patentansprüche

1. Verwendung des Wirkstoffs 3-(2-Dimethylaminomethyl-cyclohexyl)-phenol oder eines seiner pharmazeutisch verträglichen Salze zur Herstellung einer pharmazeutischen Zusammensetzung zur Behandlung von polyneuropathischem Schmerz.

2. Verwendung nach Anspruch 1, **dadurch gekennzeichnet, dass** der Wirkstoff als (1 R,2R)-3-(2-Dimethylaminomethyl-cyclohexyl)-phenol vorliegt.

3. Verwendung nach Anspruch 1 oder 2, **dadurch gekennzeichnet, dass** der Wirkstoff als Maleat vorliegt.

4. Verwendung nach einem der vorstehenden Ansprüche, **dadurch gekennzeichnet, dass** die pharmazeutische Zusammensetzung eine feste oder flüssige Darreichungsform ist.

5. Verwendung nach Anspruch 4, **dadurch gekennzeichnet, dass** die Darreichungsform zur oralen Verabreichung konfektioniert ist.

6. Verwendung nach Anspruch 4 oder 5, **dadurch gekennzeichnet, dass** die Darreichungsform zur ein-, zwei- oder dreimal täglichen Verabreichung konfektioniert ist.

7. Verwendung nach einem der Ansprüche 4 bis 6, **dadurch gekennzeichnet, dass** die Darreichungsform unter *in vitro* Bedingungen nach 1 h wenigstens 20 Gew.-% des ursprünglich in der Darreichungsform enthaltenen Wirkstoffs freisetzt.

8. Verwendung nach einem der Ansprüche 4 bis 7, **dadurch gekennzeichnet, dass** die Darreichungsform den Wirkstoff in einer Dosierung im Bereich von 15 bis 700 mg enthält, bezogen auf die freie Base.

9. Verwendung nach einem der vorstehenden Ansprüche, **dadurch gekennzeichnet, dass** der Schmerz peripherer polyneuropathischer Schmerz oder zentraler polyneuropathischer Schmerz ist.

10. Verwendung nach Anspruch 9, **dadurch gekennzeichnet, dass** der polyneuropathische Schmerz mit diabetischer Polyneuropathie oder postherpetischer Neuralgie assoziiert ist.
